(19) Europäisches Patentamt
European Patent Office
Office européen des brevets

(11) Veröffentlichungsnummer : **0 101 935**
**B1**

(12) **EUROPÄISCHE PATENTSCHRIFT**

(45) Veröffentlichungstag der Patentschrift : **07.05.86**

(51) Int. Cl.⁴ : **A 61 K 37/02**, C 07 K 15/06

(21) Anmeldenummer : **83107374.7**

(22) Anmeldetag : **27.07.83**

(54) **Verfahren zur Herstellung des C1-Inaktivators und seine Verwendung.**

(30) Priorität : **30.07.82 DE 3228502**

(43) Veröffentlichungstag der Anmeldung : **07.03.84 Patentblatt 84/10**

(45) Bekanntmachung des Hinweises auf die Patenterteilung : **07.05.86 Patentblatt 86/19**

(84) Benannte Vertragsstaaten :
**AT BE CH DE FR GB IT LI LU NL SE**

(56) Entgegenhaltungen :
**DE-A- 2 711 164**
**Chemical Abstracts Band 80, Nr. 20, 20 Mai 1974, Columbus, Ohio, USA E.F. VOGELAAR et al. "Contributions to the optimal use of human blood. III. Large-scale preparation of C1-esterase inhibitor concentrate for clinical use", Seite 275, Spalte 2, Abstract Nr. 112542m**
**Chemical Abstracts Band 79, Nr. 12, 24. September 1973, Columbus, Ohio, USA E.F. VOGELAAR et al. "Preparation of human C1-esterase inhibitor concentrate and its possible clinical use", Seite 248, Spalte 2, Abstract Nr. 70133a**
**Chemical Abstracts Band 74, Nr. 9, 1. März 1971, Columbus, Ohio, USA H. HAUPT et al. "Isolation and characterization of the C1-inactivator from human plasma", Seite 207, Spalte 1, Abstract Nr. 40613m**

(73) Patentinhaber : **BEHRINGWERKE Aktiengesellschaft**
**Postfach 1140**
**D-3550 Marburg 1 (DE)**

(72) Erfinder : **Pelzer, Hermann, Dr.**
**Ringstrasse 5**
**D-3550 Marburg-Michelbach (DE)**
Erfinder : **Heber, Helmut, Dr.**
**Am Ziegenberg 8**
**D-3550 Marburg 1 (DE)**
Erfinder : **Heimburger, Norbert, Prof. Dr.**
**Sonnenhang 10**
**D-3550 Marburg 1 (DE)**
Erfinder : **Preis, Hans Martin**
**am Wäldchen 4**
**D-3550 Marburg (DE)**
Erfinder : **Naumann, Horst**
**Herrmannstrasse 26**
**D-3550 Marburg (DE)**

(74) Vertreter : **Meyer-Dulheuer, Karl-Hermann, Dr. et al**
**HOECHST Aktiengesellschaft Zentrale Patentabteilung Postfach 80 03 20**
**D-6230 Frankfurt/Main 80 (DE)**

**EP 0 101 935 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft die Herstellung eines als C1-Inaktivator bezeichneten Proteins und dessen Verwendung zur Herstellung eines Arzneimittels.

Der C1-Inaktivator, benannt nach seiner Eigenschaft, die C1-Esterase des Komplementsystems zu inaktivieren, « kontrolliert » zusätzlich auch wichtige Enzyme der Blutgerinnung, speziell der Kontaktphase, nämlich das Präkallikrein und die Faktoren XI und XII, daneben aber auch Plasmin. Aufgrund dieser Spezifität kommt dem Cl-Inaktivator eine besondere physiologische Funktion zu. Generell kann man sagen, daß er beim Kontakt von Blut mit Oberflächen (z. B. in einer Herz-Lungen-Maschine) durch die Neutralisation der dabei entstehenden Enzyme verbraucht wird, aber auch bei Krankheitsabläufen, die zur Aktivierung der Gerinnungskaskade führen, z. B. Immunkomplexe, wie sie in Verbindung mit chronischen, vor allem rheumatischen Erkrankungen auftreten. Cl-Inaktivator ist das Medikament der Wahl bei hereditärem Angioödem.

Es gibt eine Reihe von Verfahren zur Herstellung des Cl-Inaktivators aus Humanplasma. Neben mehrstufigen Verfahren, z. B. nach Haupt, Heimburger et al. : Beitrag zur Isolierung und Charakterisierung des Cl-Inaktivators aus Humanplasma ; Eur. J. Biochem. *17,* 254-261 (1970) wird auch die Affinitätschromatographie verwendet (Reboul et al. : A simplified procedure for the purification of Cl-inaktivator from human plasma ; FEBS Letters *79,* 45 (1977)). Solche Verfahren haben gewisse Mängel : Sie sind immer noch nicht einfach genug, verlustreich und zeitaufwendig. Auch Ionenaustauscherchromatographie, evtl. kombiniert mit Gelfiltration und Affinitätschromatographie, hat nicht zu dem gewünschten Erfolg geführt. Das Verfahren nach E.F. Vogelaar u. a., Vox. Sang. 26 : 118-127 (1974), wonach Cl-Inaktivator im großen Maßstab für den klinischen Gebrauch hergestellt werden kann, entspricht nicht den heutigen Vorstellungen, die an ein solches Produkt zu stellen sind.

Es war daher die Aufgabe gestellt, Cl-Inaktivator nach einem Verfahren herzustellen, das gut reproduzierbar und mit hoher Ausbeute und Reinheit zu einem therapeutisch gut verträglichen Produkt führt.

Es wurde nun überraschenderweise gefunden, daß Cl-Inaktivator ein relativ hydrophiles Protein ist und die den Cl-Inaktivator üblicherweise begleitenden, dessen spezifische Aktivität herabsetzenden Proteine (Begleitproteine) Affinitäten zu hydrophoben Gruppen, insbesondere aromatischen Verbindungen aufweisen und daß sie somit vom Cl-Inaktivator mit Hilfe solcher Gruppen, die an einen praktisch wasserunlöslichen Träger fixiert sind, adsorbiert und davon auf diese Weise getrennt werden können. Bevorzugte aromatische Verbindungen sind Phenyl-Verbindungen. Als Träger sind die an sich bekannten Materialien geeignet, wie sie für die hydrophobe Chromatographie mit unterschiedlichen, aktiven Gruppen verwendet werden. Bevorzugt wird vernetzte Agarose, die ggfs. über einen Spacer gebundene aromatische Gruppen enthält.

Für die Isolierung des Cl-Inaktivators kommen somit Trägerverbindungen folgender Struktur in Frage :

A-B-Aro.,

wobei A den hochmolekularen, praktisch wasserunlöslichen Träger, z. B. vernetzte Agarose, vorzugsweise SEPHAROSE(R), darstellt,

B ein aliphatisches Bindeglied des aromatischen Restes ist, gebunden an diesem Träger. Auch Spacer genannte Bindeglieder sind bevorzugt,

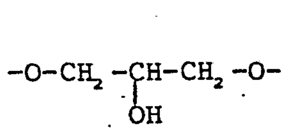 und 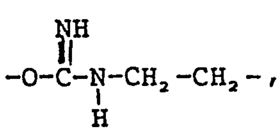,

und Aro stellt einen aromatischen Rest, vorzugsweise eine Phenylgruppe, dar.

Ein geeignetes Handelsprodukt einer AB-Aro ist PHENYL-SEPHAROSE(R) CL-4B. Bevorzugt werden diesem ähnliche Produkte, wie sie z. B. durch Umsetzung einer vernetzten Agarose, z. B. SEPHAROSE(R) 4B mit Bromcyan und anschliessend mit einem aromatischen Amin, z. B. Phenyläthylamin erhalten werden können.

Die Adsorption der Begleitproteine des Cl-Inaktivators an solche hydrophobe Träger und deren Abtrennung davon ist ein erfindungswesentlicher Schritt in dem Verfahren zur Herstellung eines Reinproduktes, dem gegebenenfalls an sich bekannte Verfahrensschritte vor- oder nachgeschaltet werden können.

Bevorzugtes Ausgangsmaterial zur Gewinnung des Cl-Inaktivators ist menschliches Plasma ; der erfindungsgemäße Verfahrensschritt kann jedoch auch auf andere, Cl-Inaktivator und Begleitproteine enthaltende wässrige Lösungen angewandt werden.

Vorteilhaft ist die Kombination des erfindungsgemäßen Verfahrensschrittes mit einem nicht

hydrophoben Adsorbens, vorzugsweise einem Ionenaustauscher, insbesondere mit Diäthylaminoäthylgruppen. Auch QAE-Gruppen enthaltende Ionenaustauscher führen ebenso wie mineralische Adsorbentien, wie beispielsweise Calciumphosphat zur Anreicherung des Cl-Inaktivators. Auch Fällungsverfahren mit Neutralsalzen, wie beispielsweise mit Ammoniumsulfat sind als Reinigungsschritte für den Cl-Inaktivator bekannt und geeignet.

Bei der therapeutischen Anwendung des Cl-Inaktivators ist wesentlich, daß sie für den Patienten gefahrlos erfolgt. Für den Ausschluß einer Hepatitisübertragung gilt die Annahme, daß über mehrere Stunden bei ca 60 °C gehaltene Proteinlösungen die Hepatitis B nicht mehr übertragen können, selbst wenn diese Lösungen vor dem Erwärmen infektiöses Hepatitis B-Virus enthielten. In einer Weiterentwicklung der vorliegenden Erfindung stellte sich deshalb die Aufgabe, das erfindungsgemäß erhaltene Produkt hepatitis-frei zur Verfügung zu stellen. Cl-Inaktivator enthaltende Lösungen können praktisch ohne Aktivitätsverlust über mehrere Stunden bei ca. 60 °C gehalten werden, wenn diese Lösungen die Aktivität des Cl-Inaktivators stabilisierende Verbindungen enthalten. Solche Verbindungen sind Aminosäuren, Zucker und/oder Zuckeralkohole oder Mischungen derselben. Ohne Aktivitätsverlust läßt sich eine Cl-Inaktivator enthaltende Lösung über 10 Stunden bei 60 °C mit einer Mischung von 2 mol/l Glycin und 60 % (w/v) Saccharose erhitzen. Allgemein werden Aminosäuren in einer Konzentration von 1-3 mol/l verwendet, Mono- und Oligosaccharide von 20-60 % (w/v) und Zuckeralkohole bis zu 75 % (w/v). Für die Erwärmung wird die Cl-Inaktivator und Stabilisator enthaltende Lösung auf einen pH-Wert zwischen 5,5-8,5, vorzugsweise 6,5-7,5, eingestellt. Neben der bevorzugt verwendeten Aminosäure Glycin sind auch folgende Aminosäuren für die Stabilisierung geeignet : L-Asparaginsäure, L-Serin, L-Valin, L-Lysin, L-Threonin, L-Tyrosin, L-Phenylalanin, L-Leucin, L-Alanin, L-Methionin, L-Prolin, L-Hydroxyprolin, L-Arginin, α- oder β-Alanin, Glutamin, α-, β- oder γ-Aminobuttersäure ; neben Saccharose folgende Zucker : Arabinose, Glucose, Galactose, Fructose, Ribose, Mannose, Rhamnose, Maltose, Raffinose ; als Zuckeralkohole sind folgende geeignet : Erythrit, Ribitol, Sorbit, Mannit.

Durch die genannten stabilisierenden Substanzen läßt sich eine Cl-Inaktivator enthaltende Lösung 1 min bis 48 Stunden bei 30 bis 100 °C erwärmen, bevorzugt im Hinblick auf die notwendige Vermeidung einer Hepatitisübertragung werden 10 Stunden und 60 °C.

Ein weiterer Gegenstand der Erfindung ist somit ein Verfahren, dadurch gekennzeichnet, daß eine den erfindungsgemäß hergestellten Cl-Inaktivator enthaltende Lösung in Gegenwert von Stabilisatoren solange erhitzt wird, bis die Lösung ihre Infektiosität, bedingt durch einen Gehalt an Hepatitis B-Virus, verliert.

Entsprechend vorgereinigtes, ggfs. über mehrere Stunden bei ca. 60 °C gehaltenes, Cl-Inaktivator und Begleitproteine enthaltendes Material mit einer Reinheit von ca. 23-25 Einheiten Cl-Inaktivator/mg (spezifische Aktivität) in wässriger Lösung wird erfindungsgemäß mit einem hydrophob-substituierten Adsorbens, A-B-Aro z. B. PHENYL-SEPHAROSE(R) behandelt. Die Adsorption der Begleitproteine des Cl-Inaktivators erfolgt bei schwach saurem, neutralem bis schwach alkalischem pH-Wert, vorzugsweise bei pH 6 pH 9. Die Leitfähigkeit der Lösung liegt zweckmäßig bei 60-120 mS. Vorteilhaft wird der Adsorptionsschritt mit dem hydrophoben Tragermaterial mit einem Fällungsschritt zur Konzentrierung, bei dem der Cl-Inaktivator aus der Lösung ausgefällt wird, kombiniert. Danach wird der ausgefällte Cl-Inaktivator mit einer wässrigen Lösung aufgenommen, die das Fällungsmittel in einer Konzentration enthält, bei welchem der Cl-Inaktivator nicht ausfällt.

Wird z. B. der Cl-Inaktivator mit einem Neutralsalz, z. B. Ammoniumsulfat präzipitiert, so kann der Niederschlag direkt im Anschluß an die Präzipitation mit einer wässrigen Lösung eines Neutralsalzes aufgenommen werden, bei deren Konzentration der Cl-Inaktivator in Lösung bleibt, z. B. einer Ammoniumsulfatkonzentration von 7-14 %. Mit einer solchen Lösung wird erreicht, daß übliche, den Cl-Inaktivator als Verunreinigung begleitende Proteine an den hydrophoben Träger gebunden werden und der Cl-Inaktivator in hoher Reinheit von dem Adsorbens, welcher die Verunreinigungen zurückhält, abgetrennt werden kann.

Für die therapeutische Anwendung wird der Cl-Inaktivator mit an sich bekannten Protein-stabilisierenden Substanzen versetzt, beispielsweise mit einer Aminosäure wie Glyzin. Das durch hydrophobe Chromatographie schließlich gereinigte Produkt wird nach Sterilfiltration auf die gewünschte, für eine Therapie wirksame Konzentration eingestellt, abgefüllt und gewünschtenfalls lyophilisiert. Die zugesetzte Aminosäure stabilisiert den Cl-Inaktivator bei der Gefriertrocknung.

Ein bevorzugtes Verfahren in einer allgemeinen Beschreibung wird beispielsweise wie folgt durchgeführt, wobei hier als Ausgangsmaterial die Grundsubstanz des Cl-Inaktivators, menschliches Plasma, zur Anwendung gelangt :

Menschliches Plasma, das z. B. Citrat enthält und frei von Kryoglobulinen und Prothrombin-Faktoren ist, wird mit einem Anionen-Austauscher behandelt und das Cl-Inaktivator-haltige Eluat mit Neutralsalzen fraktioniert. Die Begleitproteine des Cl-Inaktivators werden von den meisten anderen Plasmaproteinen mit einem hydrophoben Adsorbens adsorbiert. Somit kann mittels der hydrophoben Chromatographie mit einem einzigen Schritt der Cl-Inaktivator mit hoher Reinheit und guter Ausbeute, von Begleitproteinen abgetrennt, gewonnen werden. Der Cl-Inaktivator passiert in salzhaltiger Lösung entsprechender Konzentration eine hydrophobe Säule aus z. B. PHENYL-SEPHAROSE(R), während die meisten Begleitproteine, vor allem das in dieser Fraktion in der Regel stark angereicherte Ceruloplasmin, zurückgehalten werden.

Der CI-Inaktivator im Fällungsrückstand kann gewünschtenfalls nach Lösen in aqua dest. und Zusatz eines Zuckers, z. B. Saccharose auf 60 % (w/v) ohne wesentlichen Aktivitätsverlust 10 Std. auf 60 °C erhitzt werden. Nach Entfernung der Saccharose durch Umfällung mit Ammoniumsulfat aus verdünnter Lösung kann gleich anschließend ebenfalls mit der Technik der hydrophoben Chromatographie und in einem einzigen Schritt der CI-Inaktivator mit hoher Reinheit und guter Ausbeute, von Begleitproteinen abgetrennt, gewonnen werden.

Folgendes Beispiel erläutert die Erfindung :

Beispiel 1

Tiefgefrorenes Citratplasma, von Kryoglobulinen befreit, aus dem Faktor VIII, Cig und Human-Fibrinogen gewonnen wurden, wurde zur Gewinnung von Prothrombin-Konzentrat mit DEAE-SEPHADEX(R) gemäß EP-A-52827, EP-A-53338 und EP-A-56629 adsorbiert. Nach Abtrennung von DEAE-SEPHADEX(R) wurden dem Plasmaüberstand 10 g QAE-SEPHADEX(R) pro Liter Plasma zugesetzt und die Suspension 60 min bei 12 °C gerührt ; danach wurde das QAE-Adsorbens abgetrennt und mit 0,15 mol NaCl gewaschen.

Für die Elution wurde 1,0 mol NaCl, pH 8,0 und 0,002 5 mol/l EDTA verwendet, in einem Volumen von 0,45 l Puffer auf 10 l Plasma. Das QAE-Eluat ist eine tiefblaue Lösung, die neben Ceruloplasmin vor allem CI-Inaktivator und Faktor VII enthält. Das Eluat wurde mit flüssigem Ammoniumsulfat bei 20 °C fraktioniert : Durch Zugabe von 1 500 ml gesättigter Ammoniumsulfat-Lösung/l Eluat wurde eine 60 % Sättigung erzielt.

Der 60 % Ammoniumsulfat-Rückstand, in dem der CI-Inaktivator angereichert war, wurde anschließend der hydrophoben Chromatographie an PHENYL-SEPHAROSE(R) unterworfen. Dazu wurde die Fällung mit Aqua dest. zu einer Lösung entsprechend einer $OD_{280}$ nm ~ 55 aufgenommen, die Ammoniumsulfat-Konzentration von 7 % und der pH-Wert von 7,2 bis 7,6 eingestellt. Nach einer Klär- und Sterilfiltration wurden 1,3 l dieser Lösung über ein Gelbett mit PHENYL-SEPHAROSE(R) in Form einer Säule von 31 cm Höhe und 12 cm Breite getrennt. Der erste wasserklare Durchlauf enthielt den CI-Inaktivator, gut getrennt von dem als blaue Bande die Säule passierenden Ceruloplasmin. Die CI-Inaktivator enthaltende Fraktion wurde durch Zugabe von festem Ammoniumsulfat konzentriert : 340 g Ammoniumsulfat wurden bei 6 °C/l der durchlaufenden Fraktion zugesetzt. Die Fällung wurde abzentrifugiert und in Aqua dest. gelöst. Der Klär- und Sterilfiltration schloß sich eine Dialyse gegen einen 1,5 %igen Glyzinpuffer an. Von einer Teilmenge der Lösung wurde der Proteingehalt an CI-Inaktivator mit Hilfe der Technik der radialen Immundiffusion bestimmt und die Aktivität nach Levy und Lepow (Proc. Soc. Exp. Biol. Med. *101*, 608 (1959)) unter Verwendung von N-Acetyl-L-Tyrosinäthylester als Substrat. Die Aktivität wurde in CI-Inaktivator-Einheiten angegeben, wobei 1 E dadurch charakterisiert war, daß sie 10 E CI-Esterase inhibierte. Im Durchschnitt erhielt man Präparate mit ca. 40 E CI-Inaktivator/mg CI-Inaktivator-Protein mit einer Ausbeute von 20 %, bezogen auf das Ausgangsplasma. Das erhaltene Produkt war ca. 90 % rein, pyrogenfrei, im Tierversuch ohne Nebenwirkungen und in der Therapie des Angioödems einsetzbar.

Der 60 % Ammoniumsulfat-Fällungsrückstand konnte gewünschtenfalls mit aqua dest. gelöst, mit 60 % w/v Saccharose versetzt, auf pH 7-7,5 eingestellt und 10 h auf 60 °C erhitzt werden. Nach Abkühlen wurde die Lösung mit aqua dest. 1 : 5 verdünnt und zur Abtrennung der Saccharose durch Zugabe von gesättigter Ammoniumsulfat-Lösung bis auf 60 % Sättigung erneut gefällt. Daran schließt sich die hydrophobe Chromatographie an.

**Patentansprüche**

1. Verfahren zur Herstellung eines ggfs. hepatitis-freien, ggfs. lyophilisierbaren Inaktivators der C1-Esterase des Komplementsystems (C1-Inaktivator) durch Kombination von an sich bekannten Fällungs- und Adsorptionsverfahren, dadurch gekennzeichnet, daß als ein Adsorbens in dieser Kombination ein eine hydrophobe Gruppe, vorzugsweise eine Phenylgruppe enthaltender Träger, welcher vorzugsweise vernetzte Agarose ist, verwendet wird.

2. Verfahren zur Herstellung eines hepatitis-sicheren, durch das Verfahren des Anspruchs 1 gewonnenen C1-Inaktivator enthaltenden Arzneimittels, dadurch gekennzeichnet, daß eine den durch das Verfahren des Anspruchs 1 gewonnenen C1-Inaktivator enthaltende Lösung in Gegenwart von Stabilisatoren solange erhitzt wird, bis die Lösung ihre Infektiosität, bedingt durch einen Gehalt an Hepatitis B-Virus, verliert.

3. Verfahren nach Anspruch 2, dadurch gekennzeichnet, daß als Stabilisator eine Aminosäure, ein Zucker und/oder Zuckeralkohole oder deren Mischung verwendet wird.

4. Verfahren zur Herstellung eines den durch das Verfahren des Anspruchs 1 gewonnenen C1-Inaktivator enthaltenden Arzneimittels, dadurch gekennzeichnet, daß eine Lösung des gereinigten, ggfs. hepatitisfrei gemachten C1-Inaktivators mit einem Stabilisator versetzt und danach lyophilisiert wird.

5. Verfahren nach Anspruch 4, dadurch gekennzeichnet, daß als Stabilisator ein Zucker, vorzugsweise Saccharose, verwendet wird.

**Claims**

1. A process for the preparation of an inactivator which, if appropriate, is hepatitis-free and, if appropriate, can be lyophilized, of C1 esterase of the complement system (C1 inactivator) by combination of precipitation and adsorption processes which are known per se, which comprises using a carrier containing a hydrophobic group, preferably a phenyl group, the carrier preferably being crosslinked agarose, as an adsorbent in this combination.

2. A process for the preparation of a hepatitis-safe medicament containing C1 inactivator prepared by the process of claim 1, which comprises heating a solution containing C1 inactivator prepared by the process of claim 1 in the presence of stabilizers until the solution has lost its infectiousness caused by a content of hepatitis B virus.

3. The process as claimed in claim 2, wherein an amino acid, a sugar and/or a sugar-alcohol or a mixture thereof is used as the stabilizer.

4. A process for the preparation of a medicament containing C1 inactivator prepared by the process of claim 1, wherein a stabilizer is added to a solution of the purified C1 inactivator which, if appropriate has been rendered hepatitis-free, and the mixture is then lyophilized.

5. The process as claimed in claim 4, wherein a sugar, preferably sucrose, is used as the stabilizer.

**Revendications**

1. Procédé de préparation d'un inactivateur de l'estérase C1 du système complémentaire (inactivateur C1) éventuellement exempt de virus de l'hépatite et éventuellement lyophilisable, par combinaison d'opérations de précipitation et d'adsorption connues en soi, caractérisé en ce que l'on emploie comme adsorbant dans cette combinaison un support comportant un groupe hydrophobe, de préférence un groupe phényle, qui est de préférence de l'agar-agar réticulé.

2. Procédé de préparation d'un médicament contenant de l'inactivateur C1 obtenu par le procédé de la revendication 1 et ne présentant pas de danger d'hépatite, caractérisé en ce que l'on chauffe une solution contenant l'inactivateur C1 obtenu par le procédé de la revendication 1 en présence de stabilisants, jusqu'à ce que la solution perde son caractère infectieux, dû au virus de l'hépatite B qu'elle contient.

3. Procédé selon la revendication 2, caractérisé en ce que l'on emploie comme stabilisant un aminoacide, un sucre et/ou un sucre-alcool ou un mélange de ceux-ci.

4. Procédé de préparation d'un médicament contenant l'inactivateur C1 obtenu par le procédé de la revendication 1, caractérisé en ce que l'on mélange une solution de l'inactivateur C1 purifié, éventuellement débarrassé du virus de l'hépatite, avec un stabilisant et qu'ensuite on la lyophilise.

5. Procédé selon la revendication 4, caractérisé en ce que l'on emploie comme stabilisant un sucre, de préférence le saccharose.